# EUROPEAN PATENT APPLICATION

(11) **EP 4 321 129 A1**
(43) Date of publication of application: **14.02.2024**
(21) Application number: 22846135.6
(22) Date of filing: 13.07.2022
(51) Int. Cl.: A61C 13/02, A61C 13/00, B23K 26/352, B23K 26/362, B23K 26/364, A61L 27/06, A61C 8/00

(54) **SURFACE PROCESSING METHOD FOR DENTAL IMPLANT**

(30) Priority: 19.07.2021 KR 20210093930
(71) Applicant: Osstemimplant Co., Ltd., Gangseo-gu Seoul 07789 (KR)
(72) Inventor: PARK, Jae Jun, Gimpo-si, Gyeonggi-do 10130 (KR); RHO, Keon Ho, Incheon 21006 (KR); KIM, Kyung Il, Seoul 07791 (KR); SONG, Ju Dong, Busan 48272 (KR)
(74) Representative: Beck & Rössig European Patent Attorneys
(86) International application number: PCT/KR2022/010253
(87) International publication number: WO 2023/003265

(57) **Abstract**

Disclosed is a surface processing method for a dental implant, the surface processing method including: (a) laser-processing a surface of an implant; (b) etching the laser-processed surface of the implant; and (c) washing the etched implant, wherein a macro surface including one or more first grooves is formed at the surface of the implant by step (a), a micro surface including one or more second grooves is formed at the surface of the implant by step (b), an outer circumferential surface of the implant includes a top portion and a bottom portion, and at least one of an average width and an average depth of the first grooves is formed to be greater at the top portion of the outer circumferential surface of the implant than at the bottom portion of the outer circumferential surface of the implant.

## Description

### [Technical Field]

The present disclosure relates to a surface processing method for a dental implant.

### [Background Art]

An osseointegration characteristic means that, after an implant is fixed to bone and initial stability is achieved, permanent coupling is formed between the implant and the bone within a short treatment period.

Appropriate processing may be performed on a surface of an implant to improve the osseointegration characteristic. As a surface processing method, for example, sandblasting, anodizing, etching, or the like may be performed to chemically roughen the surface of the implant. As another surface processing method, the surface of the implant may be coated with or immersed in a material with excellent biocompatibility. An implant whose surface is processed with any of the above-described surface processing methods can shorten a post-treatment healing period.

In particular, as one example of the conventional surface processing methods, a method of performing sandblasting and etching on a surface of an implant to impart surface roughness thereto and improve the osseointegration characteristic has been proposed, but the implant whose surface is processed with this method may have a problem in that surface roughness is imparted in an irregular shape to the surface, and the osseointegration characteristic is not sufficiently implemented. In the case in which the implant with an insufficiently-implemented osseointegration characteristic is placed in the human body, complete osseointegration may not be formed between the implant and bone, and thus a treatment period may be excessively extended or a treatment failure may occur.

As described above, various surface processing methods have been conventionally proposed as methods for improving the osseointegration characteristic of an implant. Research and development are required for a surface processing method to further improve the osseointegration characteristic.

### [Disclosure]

### [Technical Problem]

The present disclosure is directed to providing a surface processing method for a dental implant that allows a dental implant to include a constant, uniformly-implemented surface roughness and have an excellent osseointegration characteristic.

### [Technical Solution]

One aspect provides a surface processing method for a dental implant, the surface processing method including: (a) laser-processing a surface of an implant; (b) etching the laser-processed surface of the implant; and (c) washing the etched implant, wherein a macro surface including one or more first grooves is formed at the surface of the implant by step (a), a micro surface including one or more second grooves is formed at the surface of the implant by step (b), an outer circumferential surface of the implant includes a top portion and a bottom portion, and at least one of an average width and an average depth of the first grooves is formed to be greater at the top portion of the outer circumferential surface of the implant than at the bottom portion of the outer circumferential surface of the implant.

In one embodiment, the one or more second grooves may be formed inside the first grooves.

In one embodiment, the average width of the first grooves may be in a range of 5 to 100 µm, and the average depth of the first grooves may be in a range of 5 to 50 µm.

In one embodiment, each of an average width and an average depth of the second grooves may be in a range of 1 to 10 µm.

In one embodiment, step (a) may include: i) laser-processing the top portion of the outer circumferential surface of the implant; and ii) laser-processing the bottom portion of the outer circumferential surface of the implant.

In one embodiment, the bottom portion of the outer circumferential surface of the implant may include a first bottom portion and a second bottom portion, and step (a) may include: i) laser-processing the top portion of the outer circumferential surface of the implant; ii) laser-processing the first bottom portion of the outer circumferential surface of the implant; and iii) laser-processing the second bottom portion of the outer circumferential surface of the implant.

Another aspect provides a dental implant which is a surface-processed dental implant in which at least a portion of the surface of the implant includes a macro surface having one or more first grooves formed thereon and a micro surface having one or more second grooves formed thereon, an outer circumferential surface of the implant includes a top portion and a bottom portion, and at least one of an average width and an average depth of the first grooves is formed to be greater at the top portion of the outer circumferential surface of the implant than at the bottom portion of the outer circumferential surface of the implant.

In one embodiment, the bottom portion of the outer circumferential surface of the implant may include a first bottom portion and a second bottom portion.

### [Advantageous Effects]

According to one aspect, it is possible to form a macro surface including one or more grooves formed in a regular shape. Also, by forming, inside the grooves, a micro surface having one or more grooves formed thereon, a surface area is improved, and simultaneously, a surface roughness is imparted in a form suitable for osseointegration. In this way, an osseointegration characteristic of a dental implant can be improved.

The advantageous effects according to one aspect of the present specification are not limited to the above-mentioned advantageous effects and should be understood as including all effects inferable from configurations described in the detailed description or claims of the present specification.

### [Description of Drawings]

FIG. 1 is a diagram showing a surface processing method for a dental implant that uses a sandblasted-and-etching technique.
FIG. 2 is a diagram showing a surface processing method for a dental implant according to one embodiment of the present specification.
FIG. 3 shows scanning electron microscope (SEM) images of a surface of a dental implant according to one embodiment of the present specification.
FIG. 4 shows SEM images of a top portion and a bottom portion of an outer circumferential surface of a dental implant of Example 3 of the present specification.
FIG. 5 shows SEM images of a top portion, a first bottom portion, and a second bottom portion of an outer circumferential surface of a dental implant of Example 4 of the present specification.
FIG. 6 is a graph showing results of measuring a coupling force for a dental implant-bone interface according to examples and comparative examples of the present specification.

### [Modes of the Invention]

Hereinafter, one aspect of the present specification will be described with reference to the accompanying drawings. However, matters mentioned in the present specification may be implemented in various different forms and thus are not limited to the embodiments described herein. Also, in the drawings, parts irrelevant to the description have been omitted to clearly describe one aspect of the present specification, and like parts are denoted by like reference numerals throughout the specification.

Throughout the specification, when a certain part is described as being "connected" to another part, this includes not only the case in which the certain part is "directly connected" to the other part, but also the case in which the certain part is "indirectly connected" to the other part with another member disposed therebetween. Also, when a certain part is described as "including" a certain element, the certain part may further include another element instead of excluding other elements unless particularly described otherwise.

When a range of a numerical value is mentioned in the present specification, the value has precision of a significant figure provided according to the standard rules in chemistry for significant figures, unless a specific range of the value is stated otherwise. For example, 10 includes a range of 5.0 to 14.9, and the number 10.0 includes a range of 9.50 to 10.49.

Hereinafter, one embodiment of the present specification will be described in detail with reference to the accompanying drawings.

### Surface processing method for dental implant

One aspect provides a surface processing method for a dental implant, the surface processing method including: (a) laser-processing a surface of an implant; (b) etching the laser-processed surface of the implant; and (c) washing the etched implant, wherein a macro surface including one or more first grooves is formed at the surface of the implant by step (a), a micro surface including one or more second grooves is formed at the surface of the implant by step (b), an outer circumferential surface of the implant includes a top portion and a bottom portion, and at least one of an average width and an average depth of the first grooves is formed to be greater at the top portion of the outer circumferential surface of the implant than at the bottom portion of the outer circumferential surface of the implant.

In dental implant treatment, an osseointegration characteristic is the most important factor. When the osseointegration characteristic is excellent, strong coupling between an alveolar bone and an implant can be formed within a short period after the implant is placed. Generally, the degree of osseointegration may vary according to a method by which a surface of an implant is processed. Examples of a surface processing method for an implant include sandblasting, anodizing, etching, or the like, and the osseointegration characteristic can be improved by appropriately applying any of the surface processing methods to impart a surface roughness to a surface of an implant. However, in an implant processed using sandblasting among the above-listed surface processing methods, since a macro surface having an irregular shape is formed, the degree of improvement of the osseointegration characteristic may be relatively insufficient.

In step (a), the surface of the implant may be laser-processed to form the one or more first grooves in a regular shape and impart a macro surface characteristic to the surface of the implant. The laser may be ultraviolet (UV) laser, and for example, a UV laser beam having a UV wavelength in a range of 200 to 400 nm may be irradiated. The UV wavelength of the UV laser beam may be 200 nm, 210 nm, 220 nm, 230 nm, 240 nm, 250 nm, 260 nm, 270 nm, 280 nm, 290 nm, 300 nm, 310 nm, 320 nm, 330 nm, 340 nm, 350 nm, 360 nm, 370 nm, 380 nm, 390 nm, 400 nm or have a range between two of these values, but is not limited thereto. Since an intensity of the UV laser beam increases with a decrease in the UV wavelength of the UV laser beam, an appropriate value may be selected within the above range in consideration of a width, a depth, and the like of the first grooves to be implemented on the surface of the implant of the present specification.

Also, a pulse width of the laser may be in a range of 1 to 200 nanoseconds (ns), and for example, may be 1 ns, 2 ns, 3 ns, 4 ns, 5 ns, 6 ns, 7 ns, 8 ns, 9 ns, 10 ns, 15 ns, 20 ns, 25 ns, 30 ns, 35 ns, 40 ns, 45 ns, 50 ns, 55 ns, 60 ns, 65 ns, 70 ns, 75 ns, 80 ns, 85 ns, 90 ns, 95 ns, 100 ns, 110 ns, 115 ns, 120 ns, 125 ns, 130 ns, 135 ns, 140 ns, 145 ns, 150 ns, 155 ns, 160 ns, 165 ns, 170 ns, 175 ns, 180 ns, 185 ns, 190 ns, 195 ns, 200 ns or have a range between two of these values, but is not limited thereto. The pulse width of the laser is a duration of the irradiated laser beam, and when the pulse width of the laser exceeds 200 ns, the width and depth of the first grooves may excessively increase, and when the pulse width of the laser is less than 1 ns, implementation of a surface roughness may not be sufficient, and the osseointegration characteristic may be degraded.

Meanwhile, the average width of the first grooves may be in a range of 5 to 100 µm, and the average depth of the first grooves may be in a range of 5 to 50 µm. For example, the average width of the first grooves may be 5 µm, 6 µm, 7 µm, 8 µm, 9 µm, 10 µm, 15 µm, 20 µm, 25 µm, 30 µm, 35 µm, 40 µm, 45 µm, 50 µm, 55 µm, 60 µm, 65 µm, 70 µm, 75 µm, 80 µm, 85 µm, 90 µm, 95 µm, 100 µm or have a range between two of these values, but is not limited thereto. When the average width of the first grooves exceeds 100 µm, the durability of the implant may be degraded, and when the average width of the first grooves is less than 5 µm, the osseointegration characteristic of the implant may be degraded.

In one example, a difference between the maximum width and minimum width of the first grooves may be 50% or less, for example, 50%, 45%, 40%, 35%, 30%, 25%, 20%, 15%, 10%, 5% or have a range between two of these values. For example, when the difference between the maximum width and minimum width is 50%, this means that, when the largest width of the first grooves is 100 µm, the smallest width is 50 µm or more. When such a characteristic is additionally satisfied, the osseointegration characteristic can be significantly improved.

Also, for example, the average depth of the first grooves may be 5 µm, 6 µm, 7 µm, 8 µm, 9 µm, 10 µm, 15 µm, 20 µm, 25 µm, 30 µm, 35 µm, 40 µm, 45 µm, 50 µm or have a range between two of these values, but is not limited thereto. When the average depth of the first grooves exceeds 50 µm, the durability of the implant may be degraded, and when the average depth of the first grooves is less than 5 µm, the osseointegration characteristic of the implant may be degraded.

In one example, a difference between the maximum depth and minimum depth of the first grooves may be 50% or less, for example, 50%, 45%, 40%, 35%, 30%, 25%, 20%, 15%, 10%, 5% or have a range between two of these values. When such a characteristic is additionally satisfied, the second grooves formed on the micro surface can be more uniformly formed, and a problem of insufficient osseointegration in some areas can be addressed.

After the one or more first grooves are formed by laser-processing and surface roughness is primarily imparted to the surface of the implant in step (a), the surface of the implant may be etched to form the one or more second grooves, having a smaller size than the first grooves, and impart a micro surface characteristic to the surface of the implant in step (b).

Each of an average width and an average depth of the second grooves may be in a range of 1 to 10 µm, and for example, may be 1 µm, 2 µm, 3 µm, 4 µm, 5 µm, 6 µm, 7 µm, 8 µm, 9 µm, 10 µm or have a range between two of these values, but is not limited thereto. When the average width and average depth of the second grooves exceeds 10 µm, the durability of the implant may be degraded, and when the average width and average depth of the second grooves is less than 1 µm, the osseointegration characteristic of the implant may be degraded.

In one example, in the implant, the one or more second grooves may be formed inside the first grooves. When the micro surface is formed inside the macro surface of the implant, coupling between the implant and bone can be improved, and a post-treatment healing period can be shortened.

A length of the implant may be in a range of 5 to 15 mm, and for example, may be 5 mm, 5.5 mm, 6 mm, 6.5 mm, 7 mm, 7.5 mm, 8 mm, 8.5 mm, 9 mm, 9.5 mm, 10 mm, 10.5 mm, 11 mm, 11.5 mm, 12 mm, 12.5 mm, 13 mm, 13.5 mm, 14 mm, 14.5 mm, 15 mm or have a range between two of these values, and the implant may have a truncated cone structure larger than a diameter of the top portion of the outer circumferential surface of the implant where screw threads are formed. The surface processing method for a dental implant may include forming the macro surface and the micro surface on the outer circumferential surface of the implant where the screw threads are formed.

In one example, at least one of the average width and average depth of the first grooves may be formed to be greater at the top portion of the outer circumferential surface of the implant than at the bottom portion of the outer circumferential surface of the implant. For example, the top portion of the outer circumferential surface may be an area where at least one of the average width and average depth is in a range of 0.5 to 2 mm, and the bottom portion of the outer circumferential surface may be the remaining area.

In one example, the top portion of the outer circumferential surface of the implant is a portion coming in contact with a cortical bone and may have the first grooves formed with a depth in a range of 1 to 15 µm and a width in a range of 1 to 25 µm, and the bottom portion of the outer circumferential surface of the implant is a portion coming in contact with a cancellous bone and may have the first grooves formed with a depth in a range of 20 to 75 µm and a width in a range of 30 to 125 µm. When relatively smaller first grooves are formed on the top portion of the outer circumferential surface, and relatively larger first grooves are formed on the bottom portion of the outer circumferential surface, the bacterial growth can be inhibited, and simultaneously, the osseointegration characteristic can be improved.

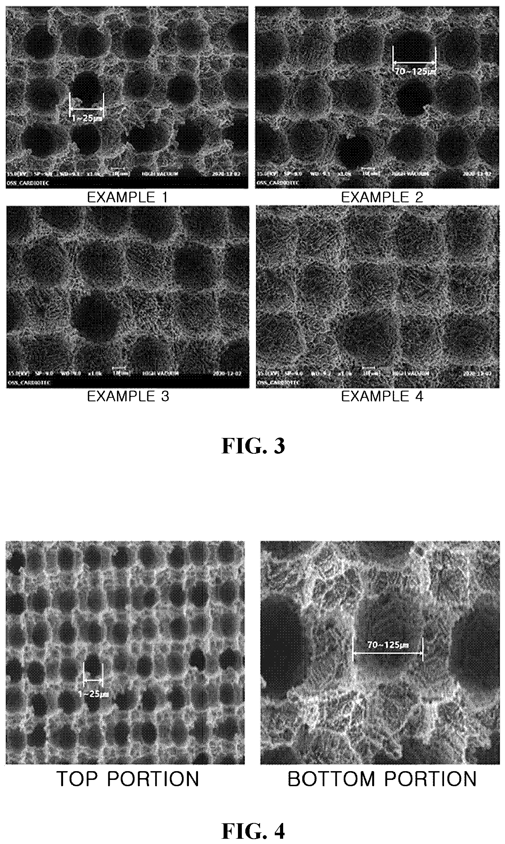

In one example, in the case in which the outer circumferential surface of the implant includes the top portion and the bottom portion, step (a) may include: i) laser-processing the top portion of the outer circumferential surface of the implant; and ii) laser-processing the bottom portion of the outer circumferential surface of the implant, and accordingly, the first grooves with an average depth in a range of 1 to 15 µm and an average width in a range of 1 to 25 µm may be formed on the top portion of the outer circumferential surface of the implant, and the first grooves with an average depth in a range of 20 to 75 µm and an average width in a range of 30 to 125 µm may be formed on the bottom portion of the outer circumferential surface of the implant. Examples of images of surface processing of the top portion and the bottom portion according to the above are found in FIG. 4.

In another example, the bottom portion of the outer circumferential surface of the implant may include a first bottom portion having an average depth in a range of 20 to 35 µm and an average width in a range of 30 to 65 µm and a second bottom portion having an average depth in a range of 40 to 75 µm and an average width in a range of 70 to 125 µm. When the bottom portion is divided into two or more areas, the inhibition of the bacterial growth and the improvement of the osseointegration characteristic can be achieved in balance.

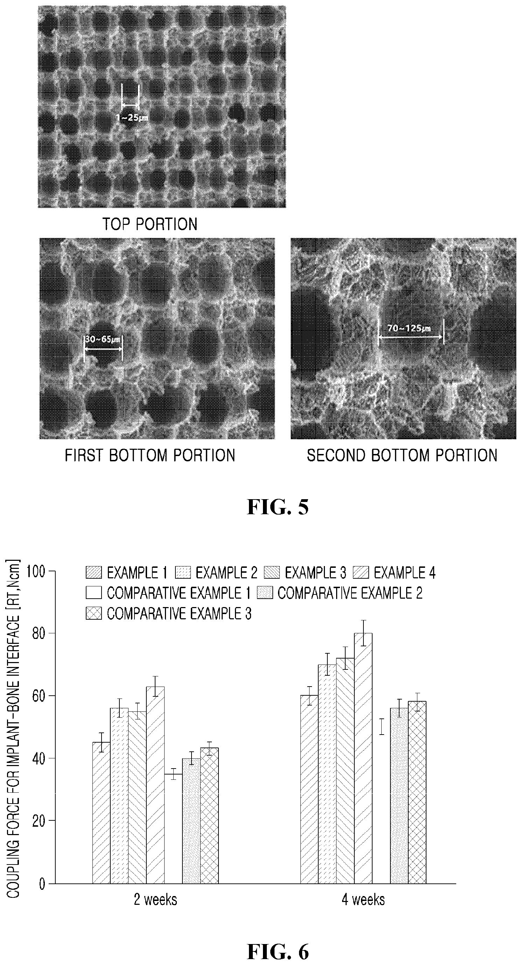

Meanwhile, in the case in which the bottom portion of the outer circumferential surface of the implant includes the first bottom portion and the second bottom portion, step (a) may include: i) laser-processing the top portion of the outer circumferential surface of the implant; ii) laser-processing the first bottom portion of the outer circumferential surface of the implant; and iii) laser-processing the second bottom portion of the outer circumferential surface of the implant, and accordingly, the first grooves with an average depth in a range of 1 to 15 µm and an average width in a range of 1 to 25 µm may be formed on the top portion of the outer circumferential surface of the implant, the first grooves with an average depth in a range of 20 to 35 µm and an average width in a range of 30 to 65 µm may be formed on the first bottom portion of the outer circumferential surface of the implant, and the first grooves with an average depth in a range of 40 to 75 µm and an average width in a range of 70 to 125 µm may be formed on the second bottom portion of the outer circumferential surface of the implant. Images of surface processing of the top portion, the first bottom portion (intermediate portion), and the second bottom portion according to the above are found in FIG. 5.

Meanwhile, the etching of step (b) may be performed by processing with an etchant, and the etchant may be one selected from the group consisting of hydrochloric acid, sulfuric acid, phosphoric acid, nitric acid, hydrogen peroxide, and a mixture of two or more thereof, but is not limited thereto. For example, in the case in which the etchant is a mixture of hydrochloric acid and sulfuric acid, content of the hydrochloric acid may be in a range of 50 to 100 vol%, and for example, may be 50 vol%, 55 vol%, 60 vol%, 65 vol%, 70 vol%, 75 vol%, 80 vol%, 85 vol%, 90 vol%, 95 vol%, 100 vol% or have a range between two of these values, but is not limited thereto.

As one non-limiting example, the etching may be performed for an amount of time in a range of 10 seconds to 120 minutes, and for example, may be performed for 10 seconds, 30 seconds, 60 seconds, 3 minutes, 5 minutes, 10 minutes, 15 minutes, 20 minutes, 25 minutes, 30 minutes, 35 minutes, 40 minutes, 45 minutes, 50 minutes, 55 minutes, 60 minutes, 65 minutes, 70 minutes, 75 minutes, 80 minutes, 85 minutes, 90 minutes, 95 minutes, 100 minutes, 105 minutes, 110 minutes, 115 minutes, 120 minutes or an amount of time in a range between two of these values. The etching time may vary according to the type of etchant. Generally, the etching time may be in a range of 10 to 40 minutes but is not limited thereto.

In step (c), the etched implant may be washed, and the etchant remaining on the surface of the implant may be removed by washing. Step (c) may be performed by immersing the etched implant in one selected from the group consisting of alcohol, purified water, and a mixture thereof, and then performing stirring or ultrasonication.

Another aspect provides a dental implant manufactured using a surface processing method for a dental implant that includes: (a) laser-processing a surface of an implant; (b) etching the laser-processed surface of the implant; and (c) washing the etched implant.

The dental implant may include, on at least a portion of a surface thereof, a macro surface having one or more first grooves formed thereon and a micro surface having one or more second grooves formed thereon, an outer circumferential surface of the implant may include a top portion and a bottom portion, and at least one of an average width and an average depth of the first grooves may be formed to be greater at the top portion of the outer circumferential surface of the implant than at the bottom portion of the outer circumferential surface of the implant.

In one example, the first grooves may be formed by laser-processing in the above-described surface processing method for an implant, and accordingly, the macro surface including the one or more first grooves may be implemented. The macro surface may be implemented in a regular, uniform shape by the laser-processing. The micro surface may be implemented by the one or more second grooves being formed inside the first grooves.

In another example, the bottom portion of the outer circumferential surface of the implant may include a first bottom portion and a second bottom portion.

In addition, details relating to the average width and average length of the first grooves and the second grooves, conditions for performing laser-processing and etching which are included in the surface processing method, and effects according thereto are the same as described above.

Meanwhile, the dental implant may be made of a titanium-based material. The titanium-based material is a material made of pure titanium or an alloy of titanium and another metal in the periodic table. For example, the titanium alloy may be at least one selected from the group consisting of titanium (Ti), aluminum (Al), silicon (Si), vanadium (V), niobium (Nb), zirconium (Zr), molybdenum (Mo), chromium (Cr), tin (Sn), tantalum (Ta), and palladium (Pd) but is not limited thereto.

As one non-limiting example, the dental implant may further include a central portion including one selected from the group consisting of a polymer, a ceramic, a metal, and a combination of two or more thereof. For example, the central portion may include the conventional titanium or an alloy thereof, a ceramic such as zirconia, or a polymer such as universal plastic, engineering plastic, super-engineering plastic, or a combination thereof, but is not limited thereto.

The surface and the central portion may be manufactured as separate parts and then combined to constitute a single dental implant, or as necessary, the surface and the central portion may be mechanically processed from a single original form and then laser-processed and/or etched to constitute a single dental implant in which the surface and the central portion are integrally formed, but the surface and the central portion are not limited thereto.

Hereinafter, examples of the present specification will be described in more detail. However, the following experimental results are only some typical experimental results of the examples, and the scope and content of the present specification should not be construed as being reduced or limited by the examples or the like. Effects of each of various implementation examples of the present specification that are not explicitly presented below will be described in detail in the corresponding parts.

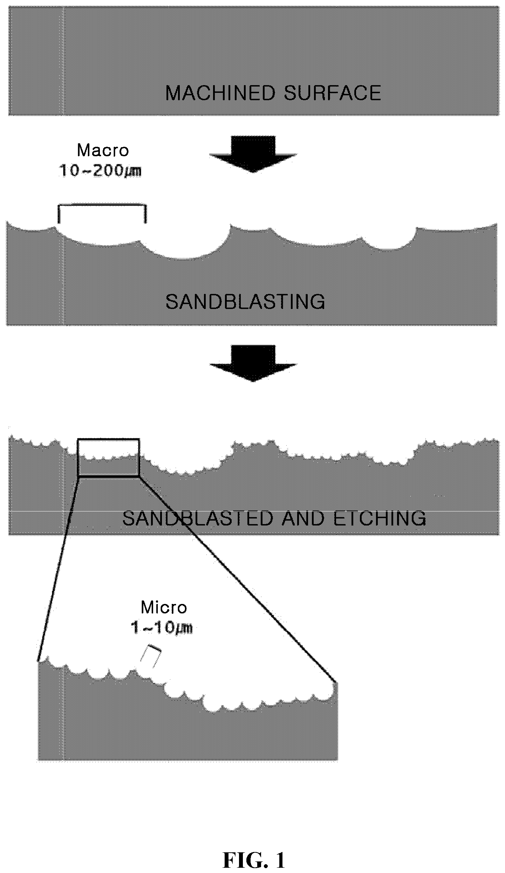

FIG. 1 is one example of the conventional surface processing method and is a diagram showing a surface processing method for a dental implant that uses a sandblasted-and-etching technique. Referring to FIG. 1, it can be confirmed that a surface roughness is imparted in an irregular shape to a surface of an implant whose surface is processed using the conventional sandblasted-and-etching technique, and in particular, it can be confirmed that a macro surface having an irregular shape is formed by sandblasting.

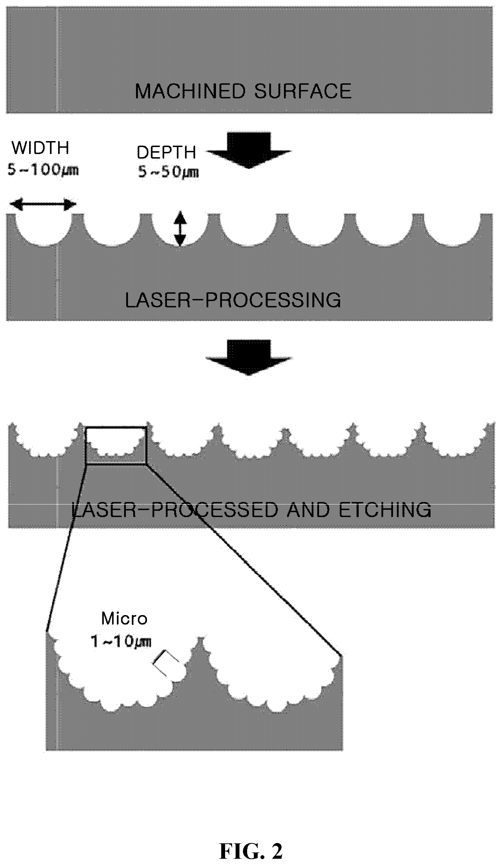

FIG. 2 is a diagram showing a surface processing method for a dental implant according to one embodiment of the present specification. Referring to FIG. 2, the surface processing method for a dental implant according to the present specification may be performed by primarily laser-processing a surface of an implant. Specifically, a machined surface of an implant to which a separate surface roughness is not imparted may be primarily laser-processed. As illustrated in FIG. 2, by laser-processing the surface of the implant and regularly forming one or more first grooves thereon, a macro surface having a uniform shape can be implemented. When a micro surface including one or more second grooves is formed by etching the macro surface having the one or more first grooves formed thereon, a surface area of the implant increases, and as a result, a coupling force for an implant-bone interface increases due to an effect of concave and convex portions, and cellular response after the implant is placed can be further activated.

### Example 1

A disk-shaped (cylindrical) Cp-Ti (Titanium Grade 4) sample having a diameter of 6 mm, a thickness of 2 mm, and a length of 10 mm was prepared, and a surface of the sample was irradiated with a UV laser beam of 355 nm for 80 ns to form a macro surface, including first grooves having an average depth in a range of 1 to 15 µm and an average width in a range of 1 to 25 µm, on the surface of the sample.

After being laser-processed, the sample was immersed for 10 minutes in a high-temperature (about 50 °C) etchant containing a mixture of hydrochloric acid and sulfuric acid to form a micro surface, including second grooves having an average width of 3 µm and an average depth of 4 µm, on the surface of the sample.

After being etched, the sample was immersed in purified water for 30 seconds, immersed in 90% ethanol and then ultrasonicated for 5 minutes, and then completely dried to manufacture a dental implant having the macro surface and the micro surface formed thereon.

### Example 2

A disk-shaped (cylindrical) Cp-Ti (Titanium Grade 4) sample having a diameter of 6 mm, a thickness of 2 mm, and a length of 10 mm was prepared, and a surface of the sample was irradiated with a UV laser beam of 355 nm for 100 ns to form a macro surface, including first grooves having an average depth in a range of 40 to 75 µm and an average width in a range of 70 to 125 µm, on the surface of the sample.

After being laser-processed, the sample was immersed for 10 minutes in a high-temperature (about 50 °C) etchant containing a mixture of hydrochloric acid and sulfuric acid to form a micro surface, including second grooves having an average width of 3 µm and an average depth of 4 µm, on the surface of the sample.

After being etched, the sample was immersed in purified water for 30 seconds, immersed in 90% ethanol and then ultrasonicated for 5 minutes, and then completely dried to manufacture a dental implant having the macro surface and the micro surface formed thereon.

### Example 3

A disk-shaped (cylindrical) Cp-Ti (Titanium Grade 4) sample having a diameter of 6 mm, a thickness of 2 mm, and a length of 10 mm was prepared, and a surface of the sample was irradiated with a UV laser beam of 355 nm for 130 ns to form a macro surface, including first grooves having an average depth in a range of 1 to 15 µm and an average width in a range of 1 to 25 µm at a top portion which is within a 1 mm-area range and first grooves having an average depth in a range of 40 to 75 µm and an average width in a range of 70 to 125 µm at a bottom portion which is the remaining area range, on the surface of the sample.

After being laser-processed, the sample was immersed for 10 minutes in a high-temperature (about 50 °C) etchant containing a mixture of hydrochloric acid and sulfuric acid to form a micro surface, including second grooves having an average width of 5 µm and an average depth of 5 µm, on the surface of the sample.

After being etched, the sample was immersed in purified water for 30 seconds, immersed in 90% ethanol and then ultrasonicated for 5 minutes, and then completely dried to manufacture a dental implant having the macro surface and the micro surface formed thereon.

### Example 4

A disk-shaped (cylindrical) Cp-Ti (Titanium Grade 4) sample having a diameter of 6 mm, a thickness of 2 mm, and a length of 10 mm was prepared, and a surface of the sample was irradiated with a UV laser beam of 355 nm for 150 ns to form a macro surface, including first grooves having an average depth in a range of 1 to 15 µm and an average width in a range of 1 to 25 µm at a top portion which is within a 1 mm-area range, first grooves having an average depth in a range of 20 to 35 µm and an average width in a range of 30 to 65 µm at a first bottom portion which is within a 4.5 mm-area range from a bottom of the top portion, and first grooves having an average depth in a range of 40 to 75 µm and an average width in a range of 70 to 125 µm at a second bottom portion which is the remaining area range, on the surface of the sample.

After being laser-processed, the sample was immersed for 10 minutes in a high-temperature (about 50 °C) etchant containing a mixture of hydrochloric acid and sulfuric acid to form a micro surface, including second grooves having an average width of 5 µm and an average depth of 5 µm, on the surface of the sample.

After being etched, the sample was immersed in purified water for 30 seconds, immersed in 90% ethanol and then ultrasonicated for 5 minutes, and then completely dried to manufacture a dental implant having the macro surface and the micro surface formed thereon.

### Comparative Example 1

A disk-shaped (cylindrical) Cp-Ti (Titanium Grade 4) sample having a diameter of 6 mm, a thickness of 2 mm, and a length of 10 mm was prepared, and a dental implant whose surface had not been processed at all was prepared.

### Comparative Example 2

A disk-shaped (cylindrical) Cp-Ti (Titanium Grade 4) sample having a diameter of 6 mm, a thickness of 2 mm, and a length of 10 mm was prepared, and aluminum oxide (Al₂O₃) powder having an average length in a range of 250 to 500 µm was sprayed for 1 minute at a pressure of 2.0 MPa to form a micro surface, including first grooves having an average depth exceeding 80 µm and an average width exceeding 150 µm, on the surface of the sample.

The subsequent etching and washing steps were performed in the same manner as in Example 3, and a dental implant having the macro surface and the micro surface formed thereon was manufactured using the conventional sandblasted-and-etching technique.

### Comparative Example 3

A disk-shaped (cylindrical) Cp-Ti (Titanium Grade 4) sample having a diameter of 6 mm, a thickness of 2 mm, and a length of 10 mm was prepared, and aluminum oxide (Al₂O₃) powder having an average length in a range of 250 to 500 µm was sprayed for 1 minute at a pressure of 2.0 MPa to form a macro surface, including first grooves having an average depth in a range of 1 to 15 µm and an average width in a range of 1 to 25 µm at a top portion which is within a 1 mm-area range and first grooves having an average depth in a range of 40 to 75 µm and an average width in a range of 70 to 125 µm at a bottom portion which is the remaining area range, on the surface of the sample, and the subsequent etching and washing steps were performed in the same manner as in Comparative Example 2 to manufacture a dental implant.

### Experimental Example 1: Surface characteristics of dental implant

FIG. 3 shows scanning electron microscope (SEM) images of surfaces of dental implants manufactured according to Examples 1 to 4 of the present specification. Referring to FIG. 3, it can be confirmed that the first grooves are regularly formed by laser-processing on the surfaces of the implants of Examples 1 to 4, and it can be confirmed that, accordingly, the macro surface is also implemented regularly and uniformly. Also, due to the second grooves being evenly formed in the first grooves, the surface roughness and surface area of the implant are improved, and the osseointegration characteristic is expected to be excellent.

### Experimental Example 2: Coupling force for dental implant-bone interface

20 of each of the dental implants manufactured according to the examples and comparative examples were implanted in the rabbit tibia by applying torque of 1 N·m. Then, after 2 weeks (14 days) and 4 weeks (28 days), torque required to remove each of the implants from each rabbit tibia was measured, and an average value thereof was obtained, which is found in FIG. 6. Referring to FIG. 6, the coupling force for an implant-bone interface was found to be somewhat insufficient in the implant of Example 1 as compared to the implants of Examples 2 to 4, the coupling force for an implant-bone interface was found to be excellent in the implants of Examples 2 to 4, and in particular, the coupling force for an implant-bone interface was found to be the best in the implant of Example 4. On the other hand, the coupling force for an implant-bone interface was found to be insufficient in Comparative Examples 1 to 3 as compared to the examples, and in particular, the coupling force for an implant-bone interface was found to be the most insufficient in the implant of Comparative Example 1 whose surface was not processed. However, in the case of Comparative Example 3, although a macro surface in which the values of the first grooves gradually increase according to the top portion and the bottom portion was formed, since only the conventional sandblasting was used, the macro surface was not uniformly formed, and the coupling force for an implant-bone interface was found to be insufficient as compared to the examples. Accordingly, it can be confirmed that, as compared to the implants of the comparative examples in which surface processing was not performed or the macro surface was formed by the conventional sandblasting, the implants of the examples have a regular, uniform macro surface formed thereon by laser-processing and thus have an improved osseointegration characteristic.

### Experimental Example 3: Bacterial growth experiment for dental implant

In order to evaluate whether the bacterial growth occurs in the dental implants manufactured according to the examples and the comparative examples, *Streptococcus mutans* IFO 13955 (*S. mutans*) was used as the bacteria, and 100 µl of *S. mutans* and 10 ml of Brain Heart Infusion (BHI) solution were mixed and then stored for 12 hours in a 37 °C, 5% CO₂ incubator for culture of the bacteria. The cultured bacteria were diluted 5 to 7 times by a factor of 10 each time using the BHI solution to obtain a concentration of 10⁶-10⁷ CFU/ml. Also, the implants were put in the bacterial solution, taken out after 4 hours, and washed with purified water, and then the degree of bacterial growth on the surface of each of the dental implants was evaluated, which is shown in Table 1 below. The relative evaluation of the coupling force for an implant-bone interface that was performed in Experimental Example 2 is also shown in Table 1.

**[Table 1]**

| Classification | Degree of bacterial growth | Coupling force for implant-bone interface |
|---|---|---|
| Example 1 | ○ | Δ |
| Example 2 | Δ | ⊚ |
| Example 3 | ⊚ | ⊚ |
| Example 4 | ⊚ | ⊚ |
| Comparative Example 1 | X | X |
| Comparative Example 2 | X | X |
| Comparative Example 3 | Δ | Δ |
| ⊚(Excellent), ○(Good), Δ(Insufficient), X(Poor) | | |

Referring to Table 1, the degree of bacterial growth of Examples 3 and 4 was found to be low, and it was confirmed that the bacterial growth hardly occurred in Examples 3 and 4 as compared to Examples 1 and 2 and Comparative Examples 1, 2, and 3. It was found that, in the case of Example 1, the degree of bacterial growth was quite low, but the coupling force for an implant-bone interface was somewhat insufficient, and in the case of Example 2, the coupling force for an implant-bone interface was excellent, but the degree of bacterial growth was somewhat high. Accordingly, it can be confirmed that, by imparting the values of the first grooves to gradually increase according to the top portion and the bottom portion or according to the top portion, the first bottom portion, and the second bottom portion as in Examples 3 and 4, the characteristic of the coupling force for an implant-bone interface can be improved, and simultaneously, the bacterial growth can be inhibited. On the other hand, in the cases of Comparative Examples 1 and 2 in which surface processing was not performed or the conventional sandblasted-and-etching technique was performed, causing the first grooves to have a depth and width that are larger than necessary, it can be confirmed that the coupling force for an implant-bone interface was poor, and the degree of bacterial growth was high as compared to the examples. However, in Comparative Example 3, the values of the first grooves were imparted to gradually increase according to the top portion and the bottom portion, and thus the bacterial growth was somewhat inhibited, and the coupling force for an implant-bone interface was somewhat better as compared to Comparative Examples 1 and 2. Still, it can be confirmed that the inhibition of the bacterial growth and the coupling force for an implant-bone interface in Comparative Example 3 were relatively insufficient as compared to Examples 3 and 4.

The above-given description of the present specification is only illustrative, and those of ordinary skill in the art to which one aspect of the present specification pertains should understand that the present specification may be easily modified to other specific forms without changing the technical spirit or essential features described in the present specification. Therefore, the embodiments described above should be understood as illustrative, instead of limiting, in all aspects. For example, each element described as a single type may be embodied in a distributed manner, and likewise, elements described as being distributed may be embodied in a combined form.

The scope of the present specification is shown by the claims below, and all changes or modifications derived from the meaning and scope of the claims and their equivalent concepts should be construed as falling within the scope of the present specification.

## Claims

1. A surface processing method for a dental implant, the surface processing method comprising:
(a) laser-processing a surface of an implant;
(b) etching the laser-processed surface of the implant; and
(c) washing the etched implant,
wherein a macro surface including one or more first grooves is formed at the surface of the implant by step (a),
a micro surface including one or more second grooves is formed at the surface of the implant by step (b),
an outer circumferential surface of the implant includes a top portion and a bottom portion, and
at least one of an average width and an average depth of the first grooves is formed to be greater at the top portion of the outer circumferential surface of the implant than at the bottom portion of the outer circumferential surface of the implant.

2. The surface processing method of claim 1, wherein the one or more second grooves are formed inside the first grooves.

3. The surface processing method of claim 1, wherein the average width of the first grooves is in a range of 5 to 100 µm, and the average depth of the first grooves is in a range of 5 to 50 µm.

4. The surface processing method of claim 1, wherein each of an average width and an average depth of the second grooves is in a range of 1 to 10 µm.

5. The surface processing method of claim 1, wherein step (a) includes:
i) laser-processing the top portion of the outer circumferential surface of the implant; and
ii) laser-processing the bottom portion of the outer circumferential surface of the implant.

6. The surface processing method of claim 1, wherein:
the bottom portion of the outer circumferential surface of the implant includes a first bottom portion and a second bottom portion; and
step (a) includes: i) laser-processing the top portion of the outer circumferential surface of the implant; ii) laser-processing the first bottom portion of the outer circumferential surface of the implant; and iii) laser-processing the second bottom portion of the outer circumferential surface of the implant.

7. A dental implant which is a surface-processed dental implant in which:
at least a portion of the surface of the implant includes a macro surface having one or more first grooves formed thereon and a micro surface having one or more second grooves formed thereon;
an outer circumferential surface of the implant includes a top portion and a bottom portion, and
at least one of an average width and an average depth of the first grooves is formed to be greater at the top portion of the outer circumferential surface of the implant than at the bottom portion of the outer circumferential surface of the implant.

8. The dental implant of claim 7, wherein the bottom portion of the outer circumferential surface of the implant includes a first bottom portion and a second bottom portion.
